# EUROPEAN PATENT APPLICATION

(11) **EP 0 748 872 A1**
(43) Date of publication of application: **18.12.1996**
(21) Application number: 95936590.9
(22) Date of filing: 08.11.1995
(51) Int. Cl.: C12N 15/81, C12N 1/19, C12N 9/30

(54) **STRAINS OF BAKERY YEAST CAPABLE OF EXPRESSING AND EXPORTING TO THE CELLULAR EXTERIOR THE ENZYME a-(1-4)-AMYLASE OF ASPERGILLUS ORYZAE: PRODUCTION PROCESS AND APPLICATION**

(30) Priority: 11.11.1994 ES 9402330
(71) Applicant: CONSEJO SUPERIOR DE INVESTIGACIONES CIENTIFICAS, E-28006 Madrid (ES)
(72) Inventor: SANZ BIGORRA Pascual Inst. Agro. y Tecn. Alimentos, Apartado Correos 73 46100 Burjasot (ES); PRIETO ALAMAN José Antonio Inst. Agro. y Tecn., Apartado Correos 73 46100 Burjasot (ES); RANDEZ GIL Francisca Inst. Agro. y Tecn. Alimentos, Apartado Correos 73 46100 Burjasot (ES)
(74) Representative: Ungria Lopez, Javier
(86) International application number: ES9500124
(87) International publication number: WO9615250

(57) **Abstract**

Production of industrial strains of bakery yeast capable of expressing and exporting to the cellular exterior the enzyme a-(1-4)-amilase of *Aspergillus oryzae*, in order to enhance the process of degradation of starch and, consequently, improve the texture and average life time of bread. To this effect, the corresponding gene has been introduced into the yeasts, and its expression is regulated by a typical yeast promoter, as is that of the actine gene *pACT1*, which is also functional in industrial growth conditions in molasses.

## Description

### Scope of the art

### Genetic engineering techniques (C12N15)

### Bakery yeast

### Indication of the state of the art

The use of the enzymes involved in the biodegradation of starch is one of the areas of major industrial interest. Strains of yeast pertaining to various types, such as, for example, *Saccharomyces cerevisiae*, are used in different industrial processes in which a partial or total degradation of different types of starches exists (production of bread, wine, beer, etc). However, since these yeasts are unable to syntherize enzymes which degrade the starch, the current practice consists in the exogenous addition of these compounds as food additives.

Thus, for example, the insufficient a-amilasic activity of the flour is counteracted up to now, in the practice of bakery, by means of the addition of malt flour and of amylolytic enzymes. The use of malt flour involves frequent problems derived from the presence of proteolytic activities which are parallel to the amilasic activities, as well as the variation in the enzymatic content depending on the lot used. It is due to this, that the use of purified amylolytic preparations are preferred [Pyler. E.J. (1988), in: Baking, Science and Technology, vol 1, pp 132-183. Sosland Publishing Co. Merriam, Kansas; Pritchard, P.E. (1992). *J. Biol. Educ.* 26. 12-18]. These additives provide the yeast with sufficient sugars for the fermentation, which improves the cellular growth and the production of gas, and modifies the rheological properties of the baking mass. Likewise, as a result of the hydrolysis of the yeast by certain a-amilases, the degree of aging of the bread (staling), is reduced, increasing the average life time of the same (Lin,W. and Lineback, D.R.(1990), *Starch* 10,385-394; Hebeda, R.E.; Bowles, L.K. and Teague, W.M. (1990), *Cereals Food World* 35, 453-457; van Dam, H.W. and Hille, J.D.R.(1992), *Cereals Food World* 37, 245-252].

The main source of the a-amilase in the baking industry is a concentrated preparation of the culture medium of the *Aspergillus Oryzae* fungus. This filament fungus, however, additionally secretes to the external environment, other enzymes (proteases, etc) which may effect in a negative way, the quality of the bread [Pyler, E.J. (1988), in: Baking, Science and Technology, vol 1, pp. 132-183. Sosland Publishing Co., Merriam, Kansas; Pritchard, P.E.(1992). *J. Biol. Educ.* 26. 12-18]. Also, its use in powder form, makes it capable of attacking the moist zones of the skin and of the mucous membranes, causing frequent irritations and alergical proceases of diverse consideration. [Pyler, E.J. (1988), in: Baking, Science and Technology, vol 1, pp.132-183. Sosland Publishing Co., Merriam, Kansas].

These problems of dosage and quality of the preparations used, makes the obtention of yeasts which are capable of expressing and secreting amylolytic enzymes to the exterior environment, of great industrial interest. Thus, the quality of the enzyme produced is normalized, avoiding the contribution of unnecessary componentes and reducing of sanitary problems which the use of these products raises.

Examples exist in literature, of the expression of amylolytic activities in strains of *Saccharomyces* *cerevisiae*, though in no case the expression and use of the enzyme a-amilase of *Aspergillus oryzae* by industrial strains of yeast is described.

### Brief description of the invention

The present invention consists in the obtention of industrial strains of bakery yeast, capable of expressing and exporting to the cellular exterior, the enzyme a-(1-4)-amilase of *Aspergillus oryzae*, in order to enhance the process of degradation of starch and consequently, improve the texture and average life time of bread. To this effect, the corresponding gene has been introduced into the yeasts and its expression is regulated by a typical yeast promoter, as is that of the actine gene (ACT1), which is also functional in industrial growth conditions in molasses.

### Detailed description of the invention

Among the amylolytic enzymes most used in industrial processes in which a degradation of the starch exists, is to be found the a-(1-4)-amilase of *Aspergillus oryzae* (Taka-amilase A). The corresponding gene consists of 8 intrones, due to which its direct expression in yeasts is impossible, since the latter lack the necessary machinary to eliminate these uncoding sequences. An alternative consists in the obtention of the corresponding cDNA parting from the specific mRNA, thus achieving a coding sequence capable of being expressed by the biosynthetic machinery of the yeasts.

The cDNA corresponding to the gene of the a-(1-4)-amilase of *Aspergillus oryzae* was obtained by means of the technique of the chain reaction of the polymerase (PCR) parting from the mRNA of *Aspergillus oryzae* grown under induction conditions (starch as the only source of carbon), using synthetic oligonucleotides specially designed for this purpose [Randez-Gil, F. and Sanz, P.(1993). *FEMS Microbiol. Letters.* 112.119-124]. This cDNA was put under the control of the constitutive promotor or the actine gene pACT1, capable of expressing itself under the industrial growth conditions in molasses.

In order to introduce the thus obtained genic constructions in the industrial yeast 10a12-13x28b4 (*trpl*)(CECT 10837), the lithium acetate procedure was followed [Ito,H.Fukuda,Y,Murata.K and Kimura,A(1983), *J.Bacteriol,* 153, 163-168], being selected in a minimum medium for the selection labeller (TRP1) present in the different plasmides.

### Description of the drawing contents

Figure 1 describes the construction of plasmides Y1pACT-AMY and YEpACt-AMY. The plasmides are represented schematically, not to scale. Initials: PCR, polymerase chain reaction: ACT1 (*sma1*) and ACT2 (*Bgl2*) are synthetic oligonucleotides containing targets *Sma1* and *Bgl2*, respectively.

Figure 2 represents the biochemical characteristics of the enzyme produced by the yeast and secreted to the culture medium. (A) optimum pH, (B) optimum temperature, (C) thermal stability after a preheating of 15 minutes at the corresponding temperatures.

Figure 3 represents the firmness curves of breads produced with a controlled industrial yeast and with the yeasts transformed with the plasmides Y1pACT-AMY and YEpACT-AMY throughout the 5 days of storage at room temperature.

### Embodiment of the invention

The cDNA corresponding to the gene of the a-(1-4)-amilase of *Aspergillus oryzae* has been previously cloned. In a recent work [Randez-Gil, F. and Sanz. P. (1993), FEMS *Microbiol. Letters* 112 119-124], this cDNA was introduced in a plasmide capable of multiplying itself both in *Escherichia coli* and in *Saccharomyces cerevisia*e and of being selected in these microorganisms thanks to the labellers Amp^{l} and UR43 respectively, resulting in plasmide pYEX-AMY.

### Construction of plasmides YIpACT-AMY and YEpACT-AMY :

As indicated in figure 1, parting from the genomic DNA of yeast *Saccharomyces cerevisiae*, and using the synthetic oligonucleotides
- ACT1 (*Sma1*):: 5'GGGACCCGGGTAAGCTGCC3' and
- ACT2 (*Bgl2*):: 5'GCGTGAAAGATCTAAAAGCTGATGTAG3'
the promotor region of the actine gene (*pACT1*) was amplified by the technique at the polymerase chain reaction (PCR). The amplified fragment was directed with the restriction enzymes *Sma1* and *Bgl2*, and the resultant fragment which contained promoter pACT1 was subcloned in plasmide pUC18 [Yanishch-Petron, C. Viera, J. and Messing, J. (1985), *Gene* 33, 103-110] previously digested with *Sma1* and *BamHl*. The resultant plasmide, pUCpACT, was sequencially digested with *Xba1*, enzyme Klenow [in the presence of deoxynucletides triphosphate (dNTPs)] and finally with *Hind3*, thus obtaining a lineal plasmide with a blunt end and a cohesive end *Hind3*.

Parting from plasmide pYEX-AMY [Randez-Gil, F. and Sanz, P.(1993),*FEMS Microbiol. Letters* 112, 119-124] a fragment containing the cDNA of the gene of the a-(1-4)-amilase of *Aspergillus oryzae* was obtained by means of sequential treatment with enzyme *EcoRl*, enzyme Klenow (in the presence of dNTPs) and enzyme *Hind3*. The fragment obtained was mixed with the open plasmide described in the previous paragraph, resulting in the plasmide pUCpACT-AMY, in which the expression of the cDNA of the a-(1-4)-amilase is controlled by promoter *pACT1*.

With the object of introducing this construction in yeasts, plasmide pUCpACT-AMY was digested with enzymes *Kpn1* and *Hind3*, obtaining a fragment which was mixed with episomal plasmide YEplac112 and integrator YIplac204 [Glatz,R.D. and Sugino,A.(1988) *Gene* 74, 527- 534] previously digested with enzymes *Kpn1* and *Hind3*, resulting in plasmides YEpACT-AMY and YIpACT-AMY respectively.

### Transformation

The aneupliode industrial yeast CECT 10837 (*Saccharomyces cerevisiae* 10a12-13x28b4, *trpl*) (Randez-Gil, F. and Sanz, P (1994). *Appl. Microbiol. biotechnol*. in the press], was transformed with integrator plasmide YIpACT-AMY, previously linearized with enzyme *EcoR5*, follow- ing the lithium acetate procedure [Ito. H., Fukuda, Y., Murata, K. and Kiumura,A. (1983), *J.Bacteriol.* 153, 163-168], selecting the transformers in a minimum medium. The linearization caused the integration to be directed towards locus *TRP1* of the chromosome of the yeast. The same procedure was used for transforming the same yeast with plasmide YEpACT-AMY.

The transformant containing plasmide YEpACT-AMY integrated, and the one containing plasmide YEpACT-AMY are found deposited, dated 26^{th} October, 1994, in the Spanish Collection of Type Cultures with numbers CECT 10838 end CECT 10839 respectively.

### Production of a-(1-4)-amilase

The previously obtained integrative and episomal transformants grow in in industrial culture medium based on beet molasses, following the increase of the biomass (Absorbance at 600 nm) and the appearance of the a-(1-4)-amilasic activity in the culture medium. Figure 2 shows that the enzyme secreted to the culture medium in both cases, has similar biochemical characteristics to that presented by the enzyme produced by *Aspergillus oryzae*: the optimum pH is approximately 5, the optimum temperature is approximately 50°C and the thermal stability of the enzyme is the same as that of the a-amilases of fungic origin (after 15 min. at 70ºC a 46% activity is lost, and after 15 min. at 80°C, the activity is completely lost). The determination of the a-(1-4)-amilasic activity was conducted using the ceralpha method, which uses p-nitrotenylmaltoheptaoside as substrate (Sheenan, H. and McCleary,B.V.(1988), *Biotechnol, Tech,* 2, 289-292]; a ceralpha unit is defined as the quantity of enzyme capable of exporting 1 mmol of p-nitrophenol per minute at 42°C under test conditions.

Table 1 represents the production of a-(1-4)-amilase by the different transformers when inoculated to flour-water mixtures. The flour (100 g) was mixed with water (250 ml) and salt (2 g), thus obtaining a conventional flour-water system. This system was inoculated with 2% (depending on the quantity of flour used), of yeasts (wet weight) and incubated 30ºC under agitation (200 rpm). An aliquot of the mixture was taken at different times and was centrifuged at 15,000 rpm during 10 min, (4ºC), subsequently analyzing the a-(1-4)-amilasic activity in the supernatante. As was to be expected, the production of the enzyme is greater in the transformants possessing the gene in high copy number.

### Effect of the production of a-(1-4)-amilase on the texture of the stored bread

Bread was produced with the different transformants following a direct process by addition of a 25% of a sponge mass in two refrigetated stages ([Barber,B. et al.(1991), *Rev. Agroq. Tecnol. Alim.* 31, 512-522]. These breads were stored at room temperature, and during different days, the texture of 2cm thick slices was measured, using an Instron 1140 press [Instron Food Testing Machine, USA]. The values of three central slices were recorded per sample, subsequently measuring the force required to compress 3/4 parts of each slice. Figure 3 represents the evolution of the texture observed for the broad produced with the industrial control yeast and the with yeasts transformed with plasmide YpACT-AMY and YEpACT-AMY. The bread produced with the transformers [YEpACT-AMY] presented less texture throughout all the times tested, no drastic increase in the firmness existing throughout the period of storage.

## Claims

1. Strains of bakery yeast, *Saccharomyces cerevisiae* 10a12-13x28b4 [YlpACT-AMY] and [YEpACT-AMY], capable of expressing and exporting to the cellular exterior, the enzyme a-(1-4)-amilase of *Aspergillus oryzae*, deposited in the Spanish Collection of Type Cultures with order numbers CECT 10838 and CECT 10839 respectively.

2. Production method of the bakery yeasts identified according to claim 1, characterized by the expression of the sequence of cDNA which contains the gene of fungus *Aspergillus oryzae* which codifies an a-(1-4)-amilasic activity, under the control of constitutive promoter *pACT1* of *Saccaromyces cerevisiae*, in strains of industrial yeast, preferably in the strain of industrial yeast *Saccharomyces cerevisiae* 10a12-13x28b4 (CECT 10837).

3. Application in the production of bread and in bakery products of bakery yeasts, according to claims 1 and 2, characterized in that it is conducted by means of inoculating recombinant strains CECT 10838 and CECT 10839 which express protein a-(1-4)-amilase of *Aspergillus oryzae*, or by the inoculation of other recombinant strains which express enzymes capable of degrading the starch, preferably the a-(1-4)-amilases, such that it contributes to improve the texture of the bread and its average life time.
